# EUROPEAN PATENT SPECIFICATION

(11) **EP 3 448 514 B1**
(45) Date of publication and mention of the grant of the patent: **29.04.2020**
(21) Application number: 17721444.2
(22) Date of filing: 28.04.2017
(51) Int. Cl.: A61N 2/02, A61N 2/00

(54) **MAGNETIC STIMULATION COIL ARRANGEMENT**
SPULENANORDNUNG FÜR MAGNETISCHE STIMULATION
AGENCEMENT DE BOBINE DE STIMULATION MAGNÉTIQUE

(30) Priority: 28.04.2016 GB 201607384
(43) Date of publication of application: 06.03.2019
(73) Proprietor: The Magstim Company Limited, Whitland, Dyfed SA34 0HR (GB)
(72) Inventor: BIGINTON, Matthew, Whitland Dyfed SA34 0HR (GB)
(74) Representative: Baker, Thomas Edward
(86) International application number: PCT/GB2017/051190
(87) International publication number: WO 2017/187184

(56) References cited:
- EP-A1- 0 977 216
- US-A1- 2004 077 923
- US-A1- 2007 027 353
- US-A1- 2015 196 772

## Description

The present invention relates to a Magnetic Stimulation (MS) coil arrangement which utilises the effect of the positioning of a ferromagnetic material to enhance the magnetic field on the patient side of the coil and reduce acoustic noise associated with the addition of ferromagnetic material.

MS coil arrangements include an apparatus for transmitting at least one pulse of current through a generally circular coil or a figure of eight coil arrangement having one or more windings, each having a plurality of turns. MS coils can be produced in a variety of shapes sizes and arrangements. Typical stimulating coils comprise an elongate conductive element wound into a coil having a plurality of turns whereby the turns are insulated from each other. When a current is passed through the wound elongate conductive element the magnetic field that is generated transfers to a patient to give a therapeutic effect or to research how various parts of the brain or body operate.

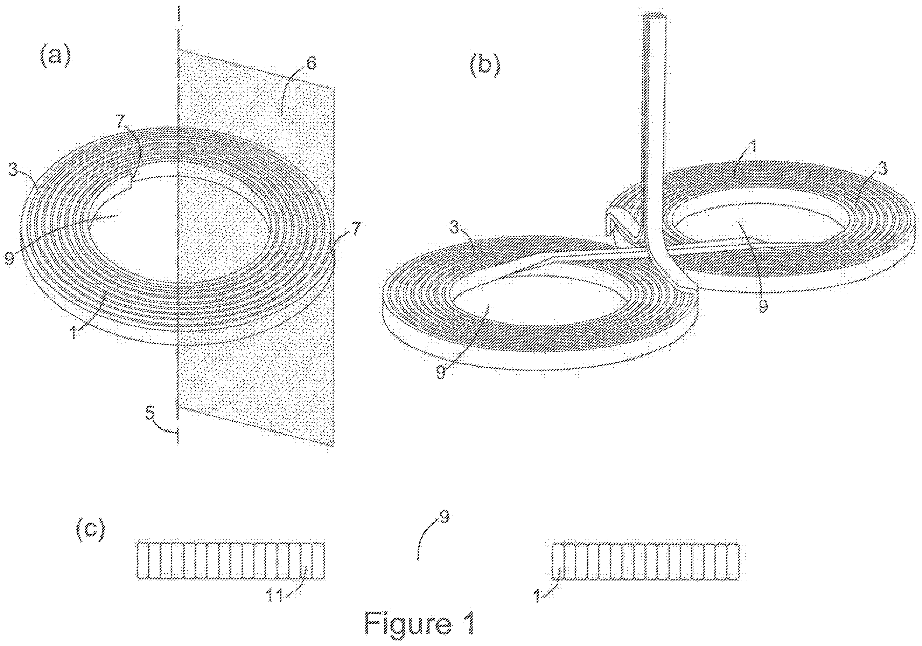

Figure 1 (a) is a three dimensional schematic representation of a circular MS coil (1) comprising a coil winding (3) having a plurality of turns. The centre (symmetry) line of the coil is marked with a dashed line (5). The coil (1) is connected to a stimulator (not shown) via the two ends of the coil winding (7). Figure 1 (b) shows a double TMS coil (or Figure of eight TMS coil) comprising two coil windings (3) to show the variety of different coils available. Each winding (3) typically has an opening (or hole) (9) at its centre. Figure 1(c) shows a cross section of the turns of a circular winding showing the individual turns (11).

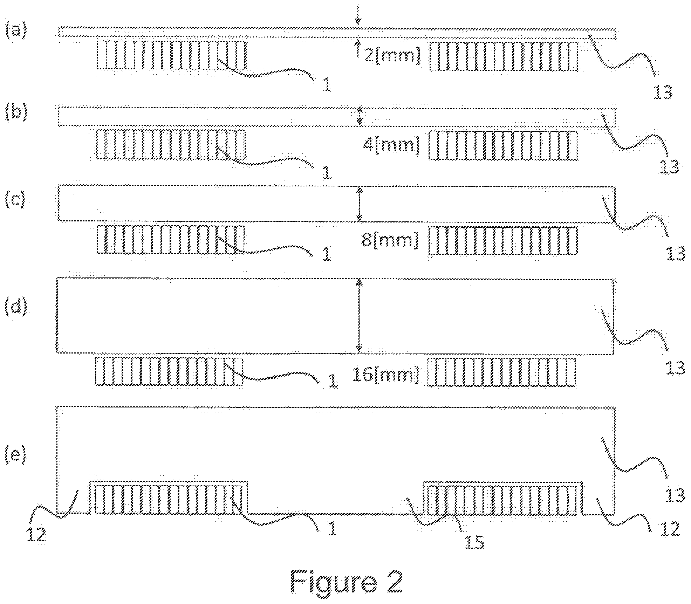

Figure 2 is a cross sectional view of the circular MS coil (1) shown in Figure 1(a) and (c) with varying sized and shaped ferromagnetic slabs (discs in this example) (13) placed on the operator (rearward) side of the coil. Figure 2 (a), (b), (c) and (d) show 2mm, 4mm, 8mm and 16mm thick slabs respectively placed on the back of the circular coil. In Figure 2 (e) the ferromagnetic material has been extended (12) into the aperture (9) of the winding and around the peripheral edges of the coil (1).

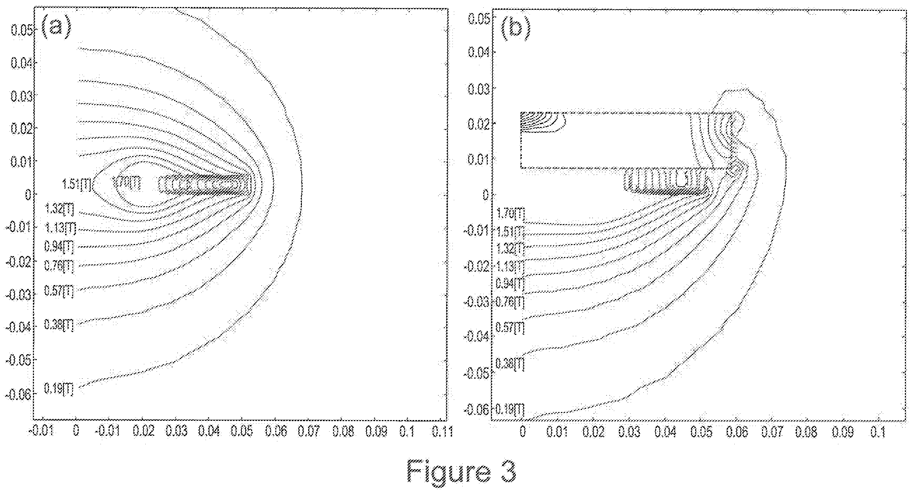

By positioning a ferromagnetic material (13) behind the coil (1) the magnetic field in comparison to the circular coil (1) alone as (illustrated by finite element modelling in Figure 3(b)) is distorted and is now asymmetric about the plane of the coil. The reluctance of the material on the rearward side of the coil (1) is now significantly lower and as a result most of the energy delivered to the coil is now stored in the volume on the forward side of the coil. This means that for the same applied energy the magnetic field strength is higher on the forward side of the coil (in the patient's tissue). Alternatively, the same magnetic field strength may be supplied to the patient whilst using less energy in comparison to the coil without the ferromagnetic material. This means that not only less energy is used to drive the coil but less joule heating occurs in the coil windings and can increase the amount of stimulations the coil can perform before overheating.

Figure 3 (a) and (b) are a comparison of the finite element modelled magnetic field contour lines around the circular MS coil (1) shown in Figure 1 (a) and the circular MS coil shown in Figure 1 (d) with a 16mm thick disc of ferromagnetic material placed adjacent its rearward side. In both cases the field lines are shown on the symmetry plane (5) illustrated in Figure 1(a). As can be clearly seen the field strength on the patient side (forward side) of the coil is increased by a significant amount. For clarity a dotted line outlines the ferromagnetic disc.

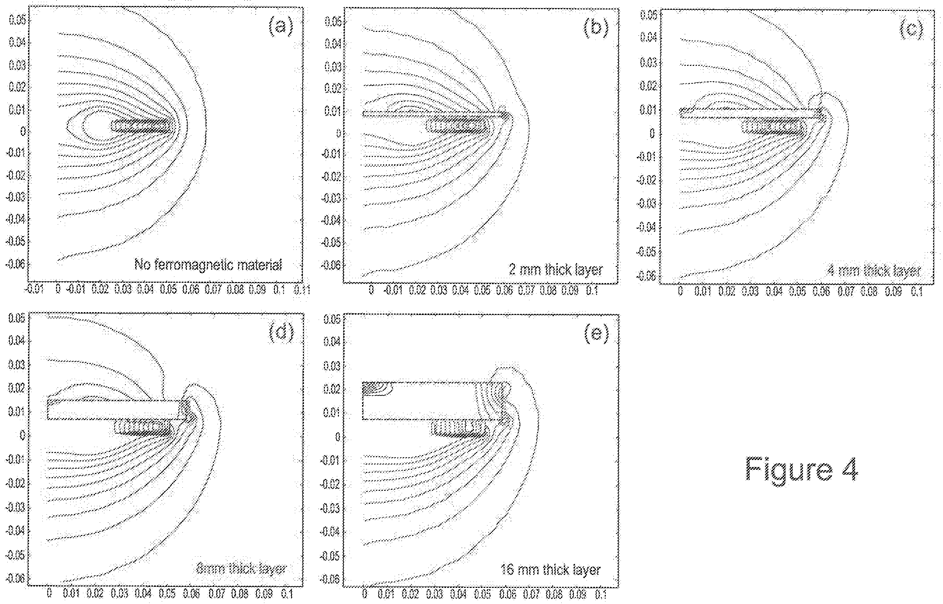

Figure 4 illustrates the effect of increasing disc thickness of the ferromagnetic material (13) from 2mm to 16mm. As can be seen from the modelled contour maps the field strength on the patient side of the coil (1) is higher in comparison to the circular coil when the 2mm disc is placed on the rearward side of the coil presented in figure 4a. Having a 4mm layer increases the field strength further (b), 8mm further again (c) and 16mm further still (d). In the case of the 16mm layer the increase has nearly levelled off and any further increase in layer thickness will have only a marginal effect on field enhancement.

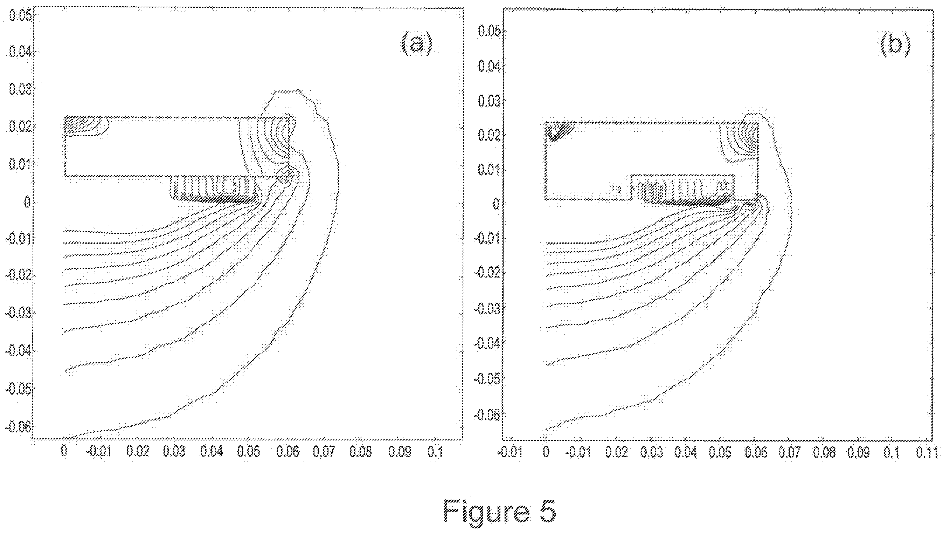

Figure 5 (a) and (b) compare the field contour lines for a thick 16mm disc of ferromagnetic material (shown in Figure 2 (d)) and the same disc albeit with the ferromagnetic material extending down into the coil winding aperture (9) and around the periphery of the coil (1).

Clearly there is a greater increase in field strength with the extended sections (15, 12) of ferromagnetic material.

Finite element results shown in Figures 3, 4 and 5 are modelled on a 2D plane in a 2D asymmetric model in COMSOL multi-physics which predicts the solutions assuming azimuthal symmetry.

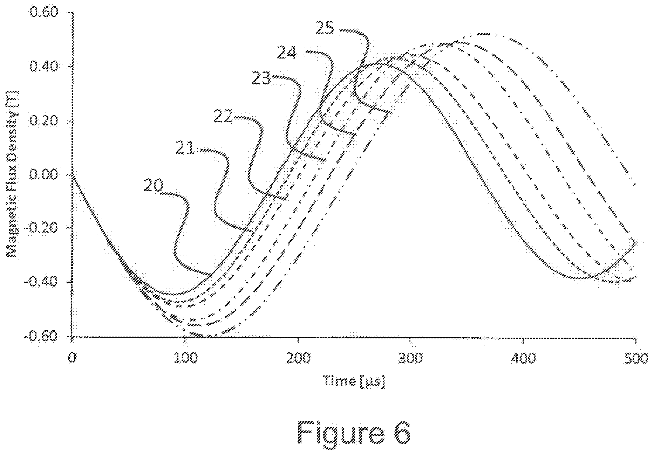

Figure 6 illustrates the magnetic field pulse waveforms from finite element modelling as recorded 2cm beneath the turns of the MS coil winding. Reference numerals 20, 21, 22, 23, 24 and 25 show the waveforms for the field distortion arrangements shown in Figure 2 (a), (b), (c), (d) and (e) respectively again illustrating that a thicker ferromagnetic materials on the back of the coil (1) produces an increased field strength at the front of the coil (1). In addition the plot clearly shows the benefit of extending the ferromagnetic material into the aperture (9) of the coil (1) around the peripheral edges.

There are problems associated with positioning a ferromagnetic material adjacent the coil at the operator's side. A solid ferromagnetic (metal) plate for example generates significant Eddy currents and quickly heats up. This significantly limits the number of pulses of current that may be supplied through the coil before the plate becomes too hot. In addition these eddy currents tend to reduce the benefits from placing a ferromagnetic metal around the coil as described in patent WO2016/005719.

US 2015/196772 A1 teaches an arrangement according to the preamble of independent claim 1.

An alternative ferromagnetic material has previously been utilised comprising grains of iron of approximately 0.1mm in diameter, each electrically insulated from an adjacent grain by a very thin inorganic insulation material. These insulated grains are then sintered and subsequently cut to the desired shape. A problem that exists with the provision of sintered encapsulated iron grains is this material, as with many other ferromagnetic materials may become saturated in particular for the case of smaller MS coils which tend to produce much higher magnetic flux densities than their larger counterparts. This means an increase in the applied external magnetic field cannot significantly increase the magnetisation of the material further so the total magnetic flux density levels off. This means that the magnetic treatment field is not as high as intended or selected as the ferromagnetic material is no longer acting as such, and thus has less effect upon the magnetic field produced. Furthermore, upon saturation the temperature of the material may increase rapidly thus meaning that operation of the magnetic stimulation arrangement must be paused. This is particularly relevant for magnetic stimulation of a patient whereby the desirable magnetic flux density may typically be as high as three Tesla at some locations near the coil.

A second problem that exists with placing electrically insulated grains as described above and solid plates of ferromagnetic material on the operator side of a TMS coil is that they tend to enhance not only the magnetic field on the patient side of the coil but also the noise generated from the magnetic stimulation coil arrangement. This is considered to be an adverse effect as it is typical for patients to have to wear ear protection with standard TMS coils and additional noise is therefore undesirable for both patient and operator.

The present invention provides an improved solution which is easily implemented, cheap, effective and in particular aids in reducing the noise produced by the magnetic stimulation coil arrangement solving the second problem with the prior art.

According to the present invention there is a magnetic stimulation coil arrangement for use in apparatus for the magnetic stimulation of tissue as defined in claim 1.

The provision of the ferromagnetic components being spaced from one another and beneficially electrically insulated from one another causes the distortion arrangement to have a lower permeability due to the provision of the spacings (effectively air gaps). This typically allows the ferromagnetic components to withstand a higher applied external magnetic field strength before saturating. The present invention typically will have a lower permeability than either solid metal or encapsulated grains of iron in the known material "Somaloy", which may saturate at a too low a flux density and which in some cases can lead to overheating. This leads to significant downtime as cooling of the distortion arrangement is required between pulses.

The ferromagnetic components are retained in the carrier through being encapsulated and/or embedded in the carrier. The carrier is a solid at room temperature and pressure.

The carrier is beneficially a matrix in which the ferromagnetic components are dispersed. The ferromagnetic components are beneficially randomly dispersed in the matrix. Dispersion of ferromagnetic components in the matrix reduces manufacturing costs whilst also providing the beneficial properties of the distortion arrangement.

The carrier is beneficially an electrical insulator and may comprise a polymer or ceramic.

The term component means a manufactured or formed object. Examples of components are ball bearings, nuts, bolts, discs, rods, cubes or parts of such objects. This improves ease and cost effectiveness of manufacture of the distortion arrangement.

The ferromagnetic components are beneficially dispersed within the carrier such that the distortion arrangement saturates at an applied field generating a flux density of greater than 1.5 Tesla.

The distortion arrangement is preferably positioned adjacent to and preferably parallel to the rearward side.

The distortion arrangement preferably comprises an array of ferromagnetic components. The ferromagnetic components are beneficially regularly spaced within the array for ease and repeatable manufacturing. However they may be randomly spaced. The ferromagnetic components are beneficially embedded or encapsulated in the carrier. The array may be an ordered array, however for ease of manufacture the ferromagnetic components in the array may be randomly positioned and oriented. This may be achieved by simply mixing the ferromagnetic components in the carrier provided in flowable form and allowing the carrier to set in a mould of the desired shape.

The distortion arrangement preferably includes a plurality of ferromagnetic components having matching dimensions for ease of manufacture. However it is appreciated that non matching dimensions or shapes will have a similar effect. The ferromagnetic components are beneficially manufactured or formed components. A plurality of the ferromagnetic components preferably have the same dimensions. The plurality of ferromagnetic components are preferably substantially identical. The provision of spherical ferromagnetic components provides a cheap and readily available ferromagnetic component that may be easily positioned in a carrier.

The maximum dimension of the ferromagnetic components is preferably between 0.045 and 1cm. The maximum dimension is preferably less than 3cm as it will be appreciated that as the size of ferromagnetic component significantly increases and progresses beyond 2cm then more significant eddy currents are induced in the ferromagnetic components thus leading to the heating of the distortion arrangement. A lower limit dimension of approximately 0.045cm is beneficial as lower than this means that it is difficult to maintain sufficient separation gap between ferromagnetic components leading to saturation at a too low a value of externally applied magnetic field strength thus reducing or removing the beneficial properties of the distortion arrangement.

The array may comprise a single layer of ferromagnetic components.

The array may comprise multiple layers of ferromagnetic components.

The carrier between the ferromagnetic components is preferably chosen to dampen the sound produced from the distortion arrangement and mechanically hold the ferromagnetic components in situ.

Typically the average spacing between ferromagnetic components may be 1/10 the size of the ferromagnetic components. However it is appreciated that by varying the gap size between the ferromagnetic components will change the effective permeability of the distortion arrangement. Therefore this average gap size could be as large as 3/1. The average gap may be as small as 1/25 in the case of 0.045cm ferromagnetic component and 1/500 in the case of the largest ferromagnetic components (3cm). It will be appreciated that there may be some touching of ferromagnetic components, particularly when mixed in size and/or shape however it is preferable that this is minimised.

The array may comprise a three dimensional array and the ferromagnetic components may be randomly spaced in the array. By providing a randomly spaced plurality of ferromagnetic components in the carrier the ferromagnetic components may be simply mixed in the carrier and formed in a mould providing the distortion arrangement.

The distortion arrangement may be arranged to correspond to the shape of the rearward side of the one or more coil windings. The distortion arrangement may comprise one or more apertures therein, each of the one or more apertures arranged to be aligned with corresponding apertures found radially inwardly of the elongate conductive element forming the one or more windings.

The or each of the coil winding preferably comprises a radially inner aperture, and the distortion arrangement may comprise a projection arranged to extend into the aperture. This has been found to further improve the effectiveness of the distortion arrangement.

The rearward side of the one or more windings comprises a peripheral edge, and the distortion arrangement may comprise a lip that extends around at least a portion of the peripheral edge. This again further improves the effectiveness of the distortion arrangement.

The ratio of the volume of ferromagnetic components to carrier material in the distortion arrangement is preferably less than 1.5:1, and preferably less than 1:1.

The present invention will now be described by way of example only with reference to the accompanying drawings in which:
Figure 1a is a three dimensional representation of a magnetic stimulation coil winding, figure 1b is a double TMS coil winding and figure 1c is a cross-section of a single magnetic stimulation coil winding.
Figure 2a-e are schematic representations of the magnetic simulation coil winding as presented in figures 1a and c with varying sized and shaped ferromagnetic disks placed on the rearward side of the coil winding.
Figure 3a and b are schematic representations of finite element modelled magnetic field contour lines around the magnetic stimulation coil winding shown in figure 1a and the magnetic stimulation coil winding as shown in figure 1d for comparative purposes.
Figure 4 is an illustration of the finite element modelled magnetic field contour lines produced by increase of the thickness of the ferromagnetic material.
Figures 5a and b are a comparison of the finite element modelled magnetic field contour lines for a 16mm disk of ferromagnetic material in comparison to the same disk having additional formations.
Figure 6 is a graphical representation of the magnetic field pulse wave forms from finite element modelling as recorded beneath the turns of MS coil winding of the distortion arrangements as presented in figures 2a-e respectively.
Figure 7 is a representation of an exemplary embodiment of the present invention where figure 7a is a representation of the patients side of a double coil winding, figure 7b is the representation of the rearward or non-patient side of a magnetic simulation coil arrangement including a distortion arrangement according to an exemplary embodiment of the present invention and figure 7c is a schematic representation of a distortion arrangement according to an exemplary embodiment.
Figure 8 is a graphical representation of the measured magnetic field on the forward or patient side of the magnetic stimulation coil windings for the exemplary embodiment as shown in figure 7.
Figure 9a-d are exemplary embodiments of ferromagnetic components for distortion arrangements according to an exemplary embodiment.
Figure 10 is a schematic representation of further ferromagnetic components for use in distortion arrangements according to an exemplary embodiment.

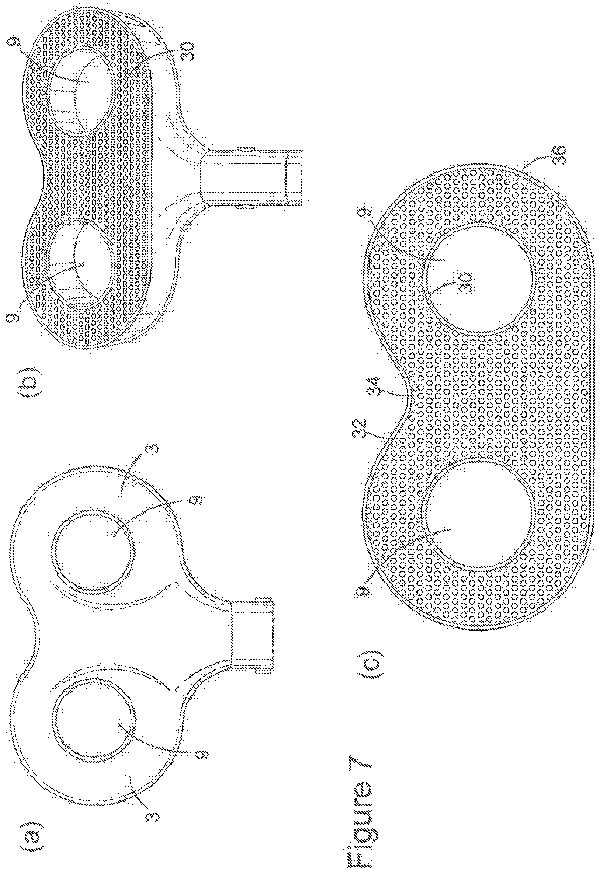

Referring to Figure 7a the forward side of the magnetic stimulation coil arrangement is represented for presenting to a patient. Represented is a double coil winding made up of two windings (3) covered to ensure generated heat does not transfer to the patient. At the rearward side as presented in Figure 7b is a distortion arrangement (30) secured to the magnetic stimulation coil windings (3). As better presented in Figure 7c, the distortion arrangement comprises a plurality of ferromagnetic components (32), shown in a carrier material (34). The carrier material (34) acts to both increase the external field strength for which the ferromagnetic component array saturates (due to the gaps) and dampens noise from the distortion arrangement. In the embodiment presented the ferromagnetic components (32) are encapsulated in the carrier material (34). In the exemplary embodiment a hexagonal array of 3mm ferromagnetic ball bearings have been provided in an array stacked two layers high. The ball bearings are separated by the carrier material (34) comprising a carrier resin and are held in place in a tray (36) which defines the outer peripheral edge and also defines the apertures (9) in the distortion arrangement (30). In this exemplary embodiment apertures (9) are presented and are aligned with the corresponding apertures in the middle of the magnetic stimulation coil windings (3). It will be appreciated, however, that the distortion arrangement (30) may be configured to cover these apertures and even more beneficially project into the apertures for increased efficiency at the forward side of the MS coil arrangement.

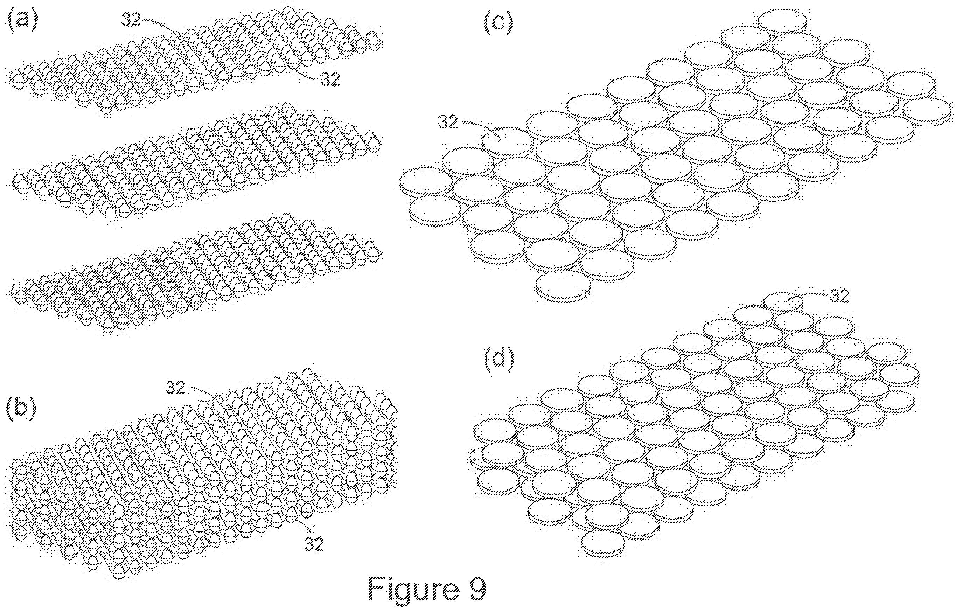

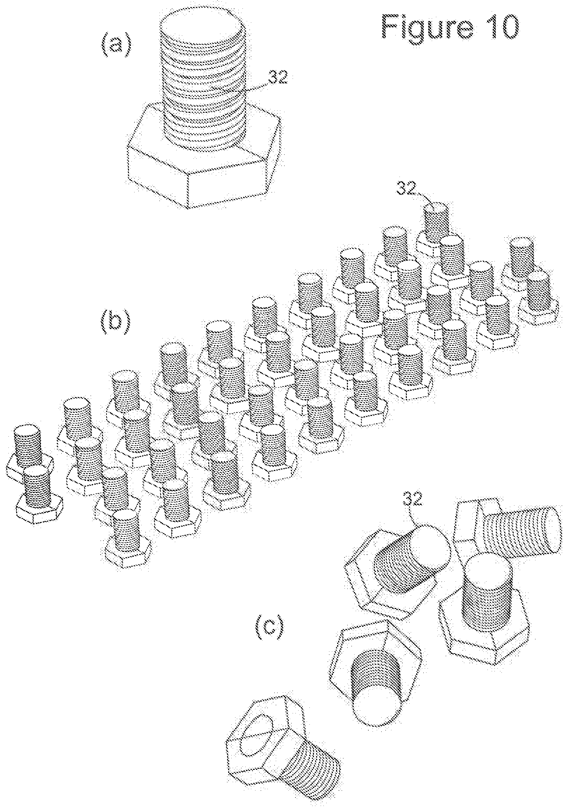

It should be noted that in the exemplary embodiment the ferromagnetic components (32) are shown as spheres or ball bearings embedded in the carrier (34). However alternative shapes of ferromagnetic components may be utilised such as discs for example shown in Figure 9 square, hexagons, triangle or any other shape. It will also be appreciated that irregular shapes may be utilised as shown in Figure 10, however for consistency of operation the ferromagnetic components (32) are preferably of consistent shape. The maximum dimension of the ferromagnetic components is preferably less than 2cm or 1/5 the diameter of the MS coil, and is preferably in the range of 0.045cm to 1cm. Such size of ferromagnetic components combined with the carrier (34) provides a distortion arrangement that can withstand a higher applied magnetic field strength before the magnetic flux density in the distortion arrangement causes the distortion arrangement to reach saturation point.

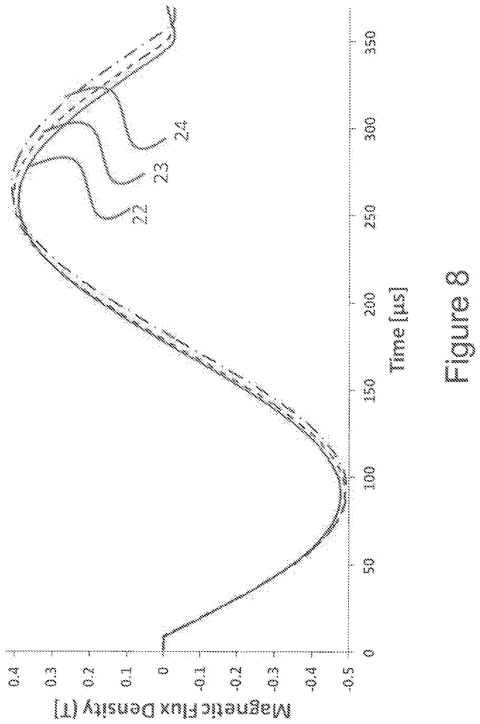

Referring now to Figure 8 there is a graphical representation of the magnetic field output over time associated with a coil with no distortion arrangement measured adjacent the forward side (as presented in curve 22) and the same measurement over time associated with a coil with the distortion arrangement comprising two full layers of ball bearings (32) and a partially filled layer positioned the rearward side (as presented in curve 24). The graph clearly shows the increase in maximum magnetic field output associated with the coil with the distortion arrangement positioned adjacent the forward side. As the number of layer of ferromagnetic components increases so does the enhancement of the field.

A further important feature of the graph presented in Figure 8 is the difference in time period associated with operation of the coil with and without a distortion arrangement. As described above, it is important that the distortion arrangement saturates at a magnetic field strength typically associated with the use of magnetic stimulation coils, and particularly transcranial magnetic stimulation coils (TMS coils). For this reason it is important that the distortion arrangement does not saturate at high applied magnetic fields. This can be checked as follows. The inductance of the coil windings (3) can be measured both with and without the distortion arrangement present at a low field strength known to be well below the saturation level. This measurement shows that the inductance of the coil increases by approximately 0.8microH. There is also an associated change in the time period. Thus, if the distortion arrangement is not saturating at a higher applied field, then the time period will be expected to be the same, as if the distortion arrangement was saturating the time period may be different to the expected change from the increased inductance and show signs of distortion from an attenuated sine wave. The reason for this is that in the event of saturation of the distortion arrangement, the distortion arrangement will stop acting as a ferromagnetic material. The distortion arrangement according to the present invention has been shown to maintain ferromagnetic properties and thus not saturate for a typical MS coil arrangement operated at full power. In addition the waveforms are clearly not distorted from the expected attenuated sine waveform which also suggests the ferromagnetic material is not being saturated.

An advantage associated with the provision of the array of ferromagnetic components embedded or encapsulated within the carrier (34) is the lower temperature rise during operation in comparison to a solid ferromagnetic plate. This is achieved through the relative spacing between the ferromagnetic components (32) in the carrier 3(4). The reduction in temperature rise within the distortion arrangement is achieved due to eddy currents being limited to only being induced within each ferromagnetic component (32) rather than through the distortion arrangement as a whole. As such, the carrier material between each ferromagnetic component (32) acts to break up and hence minimise the eddy currents.

It is also noted that extending the distortion arrangement into the centres of the coil windings and around the periphery of the coil windings may also be advantageous. This is shown in figure 2e and also enhances the field.

The present invention may be manufactured with relative ease. The ferromagnetic components may be pressed into an insulating polymeric carrier material 14, which is typically polymeric but may also be for example ceramic. A high thermally conducting potting compound may be used as the carrier material to carry heat away from the coils in addition to its other functions. The ferromagnetic components may be mixed into a fluidic carrier material which subsequently solidifies to form a flexible or rigid body. The ferromagnetic components are then dispersed in a carrier material matrix.

The present invention may be implemented into a non-planar distortion arrangement. This means that the distortion arrangement can be formed to accommodate the contours of a selected coil. For example, in a figure of eight coil comprising first and second coils each of the coils may be tilted relative to the other coil. A distortion arrangement may therefore be formed with relative ease to accommodate such a configuration due to the ease of working with ferromagnetic components having a maximum dimension of between 0.1 and 3cm, and preferably in the range 0.1 to 1cm, and even more preferably in the range between 0.4 and 1cm. Such scale of ferromagnetic components lend themselves to being moulded with a carrier material. The carrier material may comprise a potting compound, flexible rubber or other suitable non-metallic material.

The shape of the carrier material may be adjusted dependent upon the coil to be used. The shape preferably generally matches the shape of the coil.

Referring to Figures 9a to 9d alternative configurations of the ferromagnetic components in the distortion arrangement (30) are presented. In figure 9a layers of ferromagnetic components (32) are presented spaced apart for clarity purposes and in Figure 9b as show in a tightly packed array of five layers deep presented as an exemplary embodiment only as ball bearings in a hexagonal array. Figure 9c is a presentation of ferromagnetic components in the form of ferromagnetic disks (32) and Figure 9d presents ferromagnetic disks (32) two layers deep. It will be appreciated that in any embodiment the ferromagnetic components (32) are embedded, encapsulated or otherwise retained in a carrier material.

The efficacy of different shapes of ferromagnetic components (32) has been tested, and it has been found that shapes other than spherical achieve similar effects provided with the carrier material (34). For example, a distortion arrangement comprising a plurality of steel bolts as shown in Figure 10 potted in a thermosetting plastic or silicone rubber gel as the carrier (32) achieves saturation at a higher applied magnetic field.

In an embodiment according to another aspect of the invention it is possible to use, for example, iron filings or iron particles as a ferromagnetic material. However, the effectiveness of noise reduction must still be achieved and as such there must be sufficient quantity of carrier material in order to accommodate or attenuate the noise generated. For this reason the ratio of the volume of ferromagnetic particles to carrier material is less than 1.5:1, preferably less than 1:1.

Again, the particles can be mixed into a fluid carrier which is subsequently poured into a mould and solidified to form the distortion arrangement (30).

Aspects of the present invention enable enhancement of the magnetic field on the patient or forward side of the coil when placed on or adjacent to the rearward side. The increase in magnetic field strength on the patient side has been shown to be around 10%, meaning that the power supplied to the coil may now be lowered by 10% to achieve the same magnetic field output to the patient. This is achieved without the distortion arrangement saturating, and without the distortion arrangement overheating.

Aspects of the present invention have been described by way of example only and it will be appreciated by the skilled addressee that modifications and variations may be made without departing from the scope of protection afforded by the appended claims.

Aspects of the present invention enable enhancement of the magnetic field on the patient or forward side of the coil when placed on or adjacent to the rearward side. The increase in magnetic field strength on the patient side has been shown to be around 10%, meaning that the power supplied to the coil may now be lowered by 10% to achieve the same magnetic field output to the patient. This is achieved without the distortion arrangement saturating, and without the distortion arrangement overheating.

Aspects of the present invention have been described by way of example only and it will be appreciated by the skilled addressee that modifications and variations may be made without departing from the scope of protection afforded by the appended claims.

## Claims

1. A magnetic stimulation coil arrangement for use in apparatus for the magnetic stimulation of tissue, the magnetic stimulation coil arrangement comprising one or more coil windings (3) formed from an elongate conductive element and having a forward side for presentation to a patient and a rearward side, the magnetic stimulation coil arrangement further comprising a distortion arrangement (30) for distorting a magnetic field produced by the one or more coils positioned adjacent to the rearward side **characterised in that** the distortion arrangement (30) has a plurality of ferromagnetic components (32) and a carrier (34) for carrying the ferromagnetic components, the ferromagnetic components (32) being spaced from one another by the carrier (34).

2. A magnetic stimulation coil arrangement according to claim 1 wherein the carrier (34) is a matrix in which the ferromagnetic components (32) are dispersed.

3. A magnetic stimulation coil arrangement according to claim 2 wherein the ferromagnetic components (32) are randomly dispersed in the matrix.

4. A magnetic stimulation coil arrangement according to any preceding claim wherein the carrier (34) is an electrical insulator.

5. A magnetic stimulation coil arrangement according to any preceding claim wherein the distortion arrangement comprises an array of ferromagnetic components (32).

6. A magnetic stimulation coil arrangement according to any preceding claim wherein the ferromagnetic components (32) are embedded or encapsulated in the carrier (34).

7. A magnetic stimulation coil arrangement according to any preceding claim wherein the ferromagnetic components (32) include a plurality of ferromagnetic components (34) each having matching dimensions, and/or
wherein the maximum dimension of the plurality of ferromagnetic components (34) is 3cm.

8. A magnetic stimulation coil arrangement according to any preceding claim wherein the maximum dimension of the plurality of ferromagnetic components (34) is between 0.045 and 3cm.

9. A magnetic stimulation coil arrangement according to claim 5 wherein the array comprises a single layer of ferromagnetic components (34)
wherein the array comprises multiple layers of ferromagnetic components (34).

10. A magnetic stimulation coil arrangement according to any preceding claim wherein the ratio of the volume of ferromagnetic components to the carrier material in the distortion arrangement is less than 1.5:1and preferably less than 1:1.

11. A magnetic stimulation coil arrangement according to claim 6 wherein the array comprises a three dimensional array and where the ferromagnetic components (34) are randomly spaced in the array.

12. A magnetic stimulation coil arrangement according to any preceding claim wherein the distortion arrangement (30) is arranged to correspond to the shape of the rearward side of the one or more coil windings.

13. A magnetic stimulation coil arrangement according to any preceding claim wherein the distortion arrangement comprises one or more apertures therein, each of the one or more apertures arranged to be aligned with corresponding one or more apertures found radially inwardly of the elongate conductive element forming the one or more windings (3).

14. A magnetic stimulation coil arrangement according to any of claims 1-12 wherein the or each of the coil windings (3) comprises a radially inner aperture, and the distortion arrangement comprises a projection arranged to extend into the aperture.

15. A magnetic stimulation coil arrangement according to any preceding claim wherein the rearward side of the one or more windings (3) comprises a peripheral edge, and the distortion arrangement comprises a lip that extends around at least a portion of the peripheral edge.

## Patentansprüche

1. Spulenanordnung für magnetische Stimulation zur Verwendung in einer Vorrichtung für die magnetische Stimulation von Gewebe, wobei die Spulenanordnung für magnetische Stimulation eine oder mehrere Spulenwicklungen (3) umfasst, die aus einem länglichen leitfähigen Element ausgebildet sind und eine Vorderseite zur Präsentation für einen Patient und eine Rückseite aufweisen, wobei die Spulenanordnung für magnetische Stimulation weiterhin eine Verzerrungsanordnung (30) zum Verzerren eines Magnetfelds umfasst, das durch die eine oder die mehreren Spulen hergestellt wird, die der Rückseite benachbart angeordnet sind, **dadurch gekennzeichnet, dass** die Verzerrungsanordnung (30) mehrere ferromagnetische Komponenten (32) und einen Träger (34) zum Tragen der ferromagnetischen Komponenten aufweist, wobei die ferromagnetischen Komponenten (32) durch den Träger (34) voneinander beabstandet sind.

2. Spulenanordnung für magnetische Stimulation nach Anspruch 1, wobei der Träger (34) eine Matrix ist, in der die ferromagnetischen Komponenten (32) verteilt sind.

3. Spulenanordnung für magnetische Stimulation nach Anspruch 2, wobei die ferromagnetischen Komponenten (32) zufällig in der Matrix verteilt sind.

4. Spulenanordnung für magnetische Stimulation nach einem der vorhergehenden Ansprüche, wobei der Träger (34) ein elektrischer Isolator ist.

5. Spulenanordnung für magnetische Stimulation nach einem der vorhergehenden Ansprüche, wobei die Verzerrungsanordnung eine Matrix ferromagnetischer Komponenten (32) umfasst.

6. Spulenanordnung für magnetische Stimulation nach einem der vorhergehenden Ansprüche, wobei die ferromagnetischen Komponenten (32) in dem Träger (34) eingebettet oder verkapselt sind.

7. Spulenanordnung für magnetische Stimulation nach einem der vorhergehenden Ansprüche, wobei die ferromagnetischen Komponenten (32) mehrere ferromagnetische Komponenten (34) umfassen, die jeweils übereinstimmende Abmessungen aufweisen, und/oder wobei die maximale Abmessung der mehreren ferromagnetischen Komponenten (34) 3 cm ist.

8. Spulenanordnung für magnetische Stimulation nach einem der vorhergehenden Ansprüche, wobei die maximale Abmessung der mehreren ferromagnetischen Komponenten (34) zwischen 0,045 und 3 cm liegt.

9. Spulenanordnung für magnetische Stimulation nach Anspruch 5, wobei die Matrix eine einzelne Schicht ferromagnetischer Komponenten (34) umfasst, wobei die Matrix mehrere Schichten ferromagnetischer Komponenten (34) umfasst.

10. Spulenanordnung für magnetische Stimulation nach einem der vorhergehenden Ansprüche, wobei das Verhältnis des Volumens der ferromagnetischen Komponenten zu dem Trägermaterial in der Verzerrungsanordnung kleiner ist als 1,5:1 und vorzugsweise kleiner ist als 1:1.

11. Spulenanordnung für magnetische Stimulation nach Anspruch 6, wobei die Matrix eine dreidimensionale Matrix umfasst und wobei die ferromagnetischen Komponenten (34) in der Matrix zufällig beabstandet sind.

12. Spulenanordnung für magnetische Stimulation nach einem der vorhergehenden Ansprüche, wobei die Verzerrungsanordnung (30) eingerichtet ist, um der Gestalt der Rückseite der einen oder der mehreren Spulenwicklungen zu entsprechen.

13. Spulenanordnung für magnetische Stimulation nach einem der vorhergehenden Ansprüche, wobei die Verzerrungsanordnung eine oder mehrere Öffnungen darin umfasst, wobei jede der einen oder der mehreren Öffnungen angeordnet ist, um mit einer oder mehreren entsprechenden Öffnungen ausgerichtet zu sein, die radial einwärts des länglichen leitfähigen Elements zu finden sind, das die eine oder die mehreren Wicklungen (3) ausbildet.

14. Spulenanordnung für magnetische Stimulation nach einem der Ansprüche 1 bis 12, wobei die oder jede der Spulenwicklungen (3) eine radiale Innenöffnung umfasst und die Verzerrungsanordnung einen Vorsprung umfasst, der angeordnet ist, um sich in die Öffnung zu erstrecken.

15. Spulenanordnung für magnetische Stimulation nach einem der vorhergehenden Ansprüche, wobei die Rückseite der einen oder der mehreren Wicklungen (3) einen peripheren Rand umfasst und die Verzerrungsanordnung eine Lippe umfasst, die sich um mindestens einen Abschnitt des peripheren Rands herum erstreckt.

## Revendications

1. Agencement de bobine de stimulation magnétique destiné à être utilisé dans un appareil pour la stimulation magnétique d'un tissu, l'agencement de bobine de stimulation magnétique comprenant un ou plusieurs enroulements (3) de bobine formés à partir d'un élément conducteur allongé et possédant un côté avant pour une présentation à un patient et un côté arrière, l'agencement de bobine de stimulation magnétique comprenant en outre un agencement de distorsion (30) pour la distorsion d'un champ magnétique produit par la ou les bobines positionné de manière adjacente au côté arrière, **caractérisé en ce que** l'agencement de distorsion (30) possède une pluralité de composants ferromagnétiques (32) et un support (34) pour porter les composants ferromagnétiques, les composants ferromagnétiques (32) étant espacés les uns des autres par le support (34).

2. Agencement de bobine de stimulation magnétique selon la revendication 1, dans lequel le support (34) est une matrice dans laquelle sont dispersés les composants ferromagnétiques (32).

3. Agencement de bobine de stimulation magnétique selon la revendication 2, dans lequel les composants ferromagnétiques (32) sont dispersés de manière aléatoire dans la matrice.

4. Agencement de bobine de stimulation magnétique selon l'une quelconque des revendications précédentes, dans lequel le support (34) est un isolant électrique.

5. Agencement de bobine de stimulation magnétique selon l'une quelconque des revendications précédentes, dans lequel l'agencement de distorsion comprend un réseau de composants ferromagnétiques (32).

6. Agencement de bobine de stimulation magnétique selon l'une quelconque des revendications précédentes, dans lequel les composants ferromagnétiques (32) sont inclus ou encapsulés dans le support (34).

7. Agencement de bobine de stimulation magnétique selon l'une quelconque des revendications précédentes, dans lequel les composants ferromagnétiques (32) comprennent une pluralité de composants ferromagnétiques (34) ayant chacun des dimensions concordantes, et/ou
dans lequel la dimension maximale de la pluralité de composants ferromagnétiques (34) est 3 cm.

8. Agencement de bobine de stimulation magnétique selon l'une quelconque des revendications précédentes, dans lequel la dimension maximale de la pluralité de composants ferromagnétiques (34) est entre 0,045 et 3 cm.

9. Agencement de bobine de stimulation magnétique selon la revendication 5, dans lequel le réseau comprend une couche unique de composants ferromagnétiques (34)
dans lequel le réseau comprend de multiples couches de composants ferromagnétiques (34).

10. Agencement de bobine de stimulation magnétique selon l'une quelconque des revendications précédentes, dans lequel le rapport du volume des composants ferromagnétiques au matériau de support dans l'agencement de distorsion est inférieur à 1,5:1 et de préférence inférieur à 1:1.

11. Agencement de bobine de stimulation magnétique selon la revendication 6, dans lequel le réseau comprend un réseau tridimensionnel et dans lequel les composants ferromagnétiques (34) sont espacés de manière aléatoire dans le réseau.

12. Agencement de bobine de stimulation magnétique selon l'une quelconque des revendications précédentes, dans lequel l'agencement de distorsion (30) est conçu pour correspondre à la forme du côté arrière du ou des enroulements de bobine.

13. Agencement de bobine de stimulation magnétique selon l'une quelconque des revendications précédentes, dans lequel l'agencement de distorsion comporte une ou plusieurs ouvertures en son sein, chacune de la ou des ouvertures étant conçue pour être alignée sur une ou plusieurs ouvertures correspondantes trouvées radialement vers l'intérieur de l'élément conducteur allongé formant le ou les enroulements (3).

14. Agencement de bobine de stimulation magnétique selon l'une quelconque des revendications 1 à 12, dans lequel les ou chacun des enroulements de bobine (3) comprennent une ouverture radialement interne, et l'agencement de distorsion comprend une protubérance conçue pour s'étendre dans l'ouverture.

15. Agencement de bobine de stimulation magnétique selon l'une quelconque des revendications précédentes, dans lequel le côté arrière du ou des enroulements (3) comprend un bord périphérique, et l'agencement de distorsion comprend une lèvre qui s'étend autour d'au moins une partie du bord périphérique.
